Europäisches Patentamt

European Patent Office

Office européen des brevets

(19)

(11) Veröffentlichungsnummer : **0 403 959 B1**

(12)

# EUROPÄISCHE PATENTSCHRIFT

(45) Veröffentlichungstag der Patentschrift :
25.08.93 Patentblatt 93/34

(51) Int. Cl.$^5$ : **A61K 9/32, A61K 9/24**

(21) Anmeldenummer : **90111251.6**

(22) Anmeldetag : **14.06.90**

(54) **Filmbildendes wässriges Überzugsmittel für feste Arzneimittel, Verfahren zu seiner Herstellung und Verwendung.**

(30) Priorität : **20.06.89 DE 3920082**

(43) Veröffentlichungstag der Anmeldung :
**27.12.90 Patentblatt 90/52**

(45) Bekanntmachung des Hinweises auf die
Patenterteilung :
**25.08.93 Patentblatt 93/34**

(84) Benannte Vertragsstaaten :
**AT BE CH DE DK ES FR GB IT LI NL SE**

(56) Entgegenhaltungen :
**EP-A- 0 108 504**
**EP-A- 0 181 515**
**DE-A- 2 340 060**
**DE-B- 2 135 073**
**US-A- 4 452 862**

(56) Entgegenhaltungen :
**US-A- 4 606 909**
**US-A- 4 775 536**
**PATENT ABSTRACTS OF JAPAN, unexamined applications, C Field, Band 7, Nr. 11, 1983 THE PATENT OFFICE JAPANESE GOVERNMENT Seite 136 C 145**

(73) Patentinhaber : **RÖHM GMBH**
**Kirschenallee**
**D-64293 Darmstadt (DE)**

(72) Erfinder : **Petereit, Hans-Ulrich**
**Lortzingstrasse 22**
**D-6100 Darmstadt (DE)**
Erfinder : **Assmus, Manfred**
**Erbsengasse 9**
**D-6101 Bickenbach (DE)**
Erfinder : **Lehmann, Klaus, Dr.**
**Brunnersweg 12**
**D-6101 Rossdorf 1 (DE)**

EP 0 403 959 B1

## Beschreibung

Die Erfindung betrifft ein filmbildendes wäßriges Überzugsmittel für Arzneimittel, das als Bindemittel ein filmbildendes dispergiertes Polymerisat auf Basis von Acryl- und/oder Methacrylsäure oder deren Aminoalkylestern und/oder deren Alkylestern enthält. Die Eignung des Mittels zum Überziehen von Arzneimitteln setzt voraus, daß zu seiner Herstellung nur solche Bestandteile verwendet werden, die nach geltendem Erkenntnisstand pharmakologisch unbedenklich sind und wenn möglich den Anforderungen der Arzneibücher entsprechen. Die Erfindung betrifft weiterhin ein Verfahren zur Herstellung solcher Überzugsmittel sowie deren Verwendung zum Überziehen von Arzneiformen.

## Stand der Technik

Filmbildende wäßrige Überzugsmittel für Arzneimittel, die als Bindemittel dispergierte Polymerisate auf Basis von Acryl- und/oder Methacrylsäure oder deren Aminoalkylestern und/oder deren Alkylestern enthalten, haben sich in der Arzneimittelherstellung seit vielen Jahren bewährt. Vor den früher an ihrer Stelle verwendeten organischen Polymerisatlösungen haben wäßrige Dispersionen den Vorteil, daß bei ihrer Anwendung nur Wasserdampf entweicht, der ohne weiteres in die Atmosphäre entlassen werden kann, während die Dämpfe von organischen Polymerisatlösungen durch aufwendige Methoden zurückgewonnen oder vernichtet werden müssen.

Die Oberflächen von Arzneimitteln, die mit filmbildenden Überzugsmitteldispersionen beschichtet werden, durchlaufen beim Trocknen eine Phase, in der die Filmhülle noch mechanisch instabil und klebrig ist und die beschichteten Arzneimittelkörper zum Zusammenkleben neigen. Dabei kann der ganze Ansatz verklumpen oder es werden durch Berührung mit anderen Körpern Teile der Beschichtung aus dem Überzugsfilm herausgerissen. Solche Fehlstellen führen zu einem unerwünschten Freisetzungsverhalten des Wirkstoffes, der in dem überzogenen Arzneimittel enthalten ist.

Um diese klebrige Phase ohne Nachteil für die Produkteigenschaften zu überwinden, ist es allgemein gebräuchlich, der Überzugsmitteldispersion Gleit- oder Trennmittel zuzusetzen. Das sind feinteilige Feststoffe, insbesondere Silikate, wie Talkum oder feinteilige Kieselsäure. Letztere gelten als pharmakologisch unbedenklich, werden aber trotzdem wegen vermuteter Risiken von manchen Arzneimittelherstellern zunehmend gemieden. Diese Gleit- oder Trennmittel lassen sich in dem wäßrigen Überzugsmittel nicht dauerhaft dispergieren. Daher müssen Überzugsmitteldispersionen kurz vor der Anwendung frisch zubereitet und während des Sprühens ständig gerührt werden.

Die Beschichtung von Arzneiformen ohne solche die Klebrigkeit verhindernden Zusatze erfordert großes Geschick und eine sehr langsame Arbeitsweise. In der DE-OS 23 40 060 ist ein Verfahren beschrieben, bei dem Weichgelatinekapseln im Wirbelschichtverfahren zuerst mit einer organischen Überzugslösung und anschließend mit einer wäßrigen Überzugsdispersion beschichtet werden. Die letztere enthält keine pulverförmigen Zusätze zur Verhinderung des Verklebens und muß daher sehr behutsam aufgesprüht werden. Das verhältnismäßig niedrige Raumgewicht der Kapseln erleichtert die Beschichtung, da die Kapseln in der Wirbelschicht weniger als kompakte Arzneiformen zum Verkleben neigen.

Die gemäß DE-OS 23 40 060 eingesetzten Überzugsmitteldispersionen können weichmachende Zusätze enthalten, wie Glykole, Polyglykole, Glycerin, Triacetin, ethoxylierte Partialglyceride mittelkettiger Fettsäuren. Auch Glycerin-monooelat wird als geeigneter Weichmacher genannt. Dieses tensidartig aufgebaute Produkt hat je nach der Herstellungsweise einen HLB-Wert von 2,6 bis 4 und ist in Wasser nicht löslich und nicht selbstemulgierend.

EP-A-18 15 15 beschreibt Arzneimittel überzüge, die neben einem filmbildenden dispergierten Polymerisat einen Weichmaches enthalten können.

## Aufgabe und Lösung

Der Erfindung liegt die Aufgabe zugrunde, ein aus pharmakologisch unbedenklichen Bestandteilen aufgebautes filmbildendes wäßriges Überzugsmittel für Azneimittel zu schaffen, das mit oder ohne Gehalt von Pigmenten oder Fullstoffen in gebrauchsfertiger Form lagerfähig ist und ohne Zusatz von Gleit- und Trennmitteln, wie Talkum, Kieselsäure oder anderen klebverhindernden Feststoffzusätzen, ohne Verklebungsprobleme mit üblichen Auftragsverfahren auf feste Arzneiformen aufgebracht werden kann.

Die neuen Überzugsmittel gemäß der Erfindung enthalten
a) ein filmbildendes dispergiertes Polymerisat auf Basis von Acryl- und/oder Methacrylsäure oder deren Aminoalkylestern und/oder deren Alkylestern,
b) einen wasserunlöslichen organischen lipophilen Emulgator mit einem HLB-Wert zwischen 3,5 und 7.

Die genannten lipophilen Emulgatoren haben die überraschende Wirkung, daß sie das Verkleben der Arzneiformen während des Beschichtungsverfahrens verhindern. Weiterhin wirken sie einer eventuellen Neigung der fertigen Arzneiformen zum Verkleben unter ungünstigen Lagerungsbedingungen entgegen. Um diese Wirkung zu verstärken, kann am Schluß des Beschichtungsverfahrens ein dünner Film des lipophilen Emulgators aus einer wäßrigen Dispersion, die sonst keine weiteren Überzugsmittel enthält, auf die Arzneiformen aufgebracht werden.

Es wird vermutet, daß sich die lipophilen Emulgatoren beim Trocknen von dem Überzugsmittel trennen und an der Oberfläche des Überzugs eine trennende Grenzschicht mit verminderter Haftwirkung ausbilden. Dadurch sind Zusätze von nicht stabil dispergierten Feststoffen, vor allem Talkum, entbehrlich. Andererseits wirken die lipophilen Emulgatoren dispergierend auf übliche Pigmente und Füllstoffe, so daß durch deren Mitverwendung die Lagerfähigkeit nicht eingeschränkt wird.

Der lipophile Emulgator

In dem beanspruchten Bereich des HLB-Wertes sind die lipophilen Emulgatoren in wäßrigen Dispersionen der erwähnten Polymerisate stabil dispergierbar. Für lipophile Emulgatoren mit einem niedrigeren HLB-Wert gilt das nicht; sie trennen sich beim Lagern von dem Überzugsmittel ab. Liegt der HLB-Wert über 7, so ist das Emulgiermittel wasserlöslich und die klebverhindernde Wirkung tritt nicht auf. Der bevorzugte Bereich des HLB-Wertes liegt bei 3,5 bis 5,0.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444-448; weitere Literaturhinweise in Römpp, Chemie-Lexikon, 8.Aufl. 1983, S.1750.

Typische Vertreter der erfindungsgemäß einzusetzenden lipophilen Emulgatoren sind in der nachfolgenden Liste (einschließlich ihrer Handelsbezeichnungen) zusammengestellt:

| Chemische Bezeichnung | HLB-Wert | Handelsname |
|---|---|---|
| Ethylenglykol-monolaurat | 3,6 | Cithrol EGML |
| Diethylenglykol-monooleat | 5,0-5,7 | Cithrol DGMO |
| Diethylenglykol-monostearat | 4,4-5,0 | Cithrol DGMS |
| Diethylenglykol-distearat | 5,5 | Cithrol DGDS |
| Propylenglykol-monolaurat | 3,6 | Cithrol PGML |
| Propylenglykol-monooleat | 3,9 | Cithrol PGMO |
| Propylenglykol-ricinoleat | 3,6 | Cithrol PGMR |
| Propylenglykol-monostearat | 3,2 | Cithrol PGMS |
| Dipropylenglykol-monooleat | 5,3-6,0 | Cithrol DPGMO |
| Glycerin-monostearat | 3,5-3,8 | Abracol, Cutina GMS |
| Glycerin-distearat | 3,4-4,2 | Cithrol GDS |
| Glycerin-monolaurat | 4,9-5,6 | Cithrol GMR |
| Glycerin-monoricinoleat | 3,6 | Cithrol GMO |
| Sorbitan-sesquioleat | 3,7 | Arlacel C |
| Sorbitan-monooleat | 4,3 | Atlas G-4884 |
| Sorbitan-monostearat | 4,7 | Span 80, Atpet |
| Sorbitan-monoisostearat | 4,7 | Span 60, Crill 6 |
| Polyethylenglykol-200-dilaurat | 6,0 | Cithrol 2DL |

| Chemische Bezeichnung | HLB-Wert | Handelsname |
|---|---|---|
| Polyethylenglykol-300-diricinoleat | 5,0 | Cithrol 3DR |
| POE-(2)-cetylalkohol | 5,3 | Brij 52 |
| POE-(2)-stearylalkohol | 4,9 | Brij 72 |
| POE-(2)-oleylalkohol | 4,9 | Brij 92 |
| POE-cetyl-oleylalkohol | 6,0 | Atlas G-70140 |
| POE-(6)-sorbitan-Bienenwachs-derivat | 5,0 | Atlas G-1702 |
| POE-(20)-sorbitan-Bienen-wachsderivat | 5,0 | Atlas G-1726 |

Die im Handel angebotenen Produkte entsprechen in ihrer chemischen Zusammensetzung oft nicht genau der angegebenen Bezeichnung. Abgesehen davon, daß es sich bei teilveresterten Polyalkoholen meist um Isomerengemische handelt, sind sie in der Regel mit mehr oder weniger großen Anteilen von höher oder niedriger veresterten Alkoholen vermischt. Beispielsweise enthalten die als Monoglyceride von Fettsäuren gehandelten Produkte Anteile an freiem Glycerin und an Di- und Triglyceriden sowie an freien Fettsäuren; diese Anteile können bis zu 50 % des Produktes ausmachen und haben einen entsprechend großen Einfluß auf den HLB-Wert. So finden sich für Produkte, die als Glycerin-monooleat angeboten werden, HLB-Werte zwischen 2,7 und 4. Die Eignung eines bestimmten lipophilen Emulgators für die Zwecke der Erfindung hängt daher weniger von seiner chemischen Bezeichnung als von dem gemessenen HLB-Wert ab.

Der Anteil des lipophilen Emulgators liegt vorzugsweise bei 0,1 bis 10 Gew.-%, bezogen auf das Gewicht des Polymerisats.

Wenn das Überzugsmittel Pigmente oder Füllstoffe enthält, ist ein Gehalt zwischen 2 und 10 Gew.-% vorteilhaft. Mengen über 10 Gew.-% können die Haftung des Polymerisatfilms beeinträchtigen und zu ungleichmäßigen Überzügen führen.

Das dispergierte Polymerisat

Dispersionen von Polymerisaten auf Basis von Acryl- und/oder Methacrylsäure und/oder deren Alkylestern, die aus wäßriger Dispersion filmbildend sind, haben sich als Überzugsmittel für feste Arzneiformen bewährt. Aus den DE-PS 16 17 351 und 18 14 669 sind Dispersionen wasserunlöslicher Emulsionspolymerisate bekannt, deren Filme durch Zusätze wasserlöslicher Stoffe für Wirkstoffe durchlässig gemacht werden. Ohne solche Zusätze sind Filme aus Emulsionspolymerisaten gemäß DE-PS 21 35 073, die neben Einheiten von Acryl- und/oder Methacrylsäureestern funktionelle Einheiten mit Carboxylgruppen enthalten, im Darmsaft löslich. Ersetzt man die Carboxylgruppen durch Aminogruppen, so erhält man magensaftlösliche Überzüge. Durch Abmischungen von filmbildenen Dispersionen, die wasserunlösliche Emulsionspolymerisate enthalten, mit solchen von alkalilöslichen, carboxylgruppenhaltigen oder von säurelöslichen, aminogruppenhaltigen Emulsionspolymerisaten lassen sich gemäß DE-OS 34 05 378 oder 35 24 337 Arzneimittelüberzüge mit genau definiertem Freisetzungsverhalten für den eingeschlossenen Wirkstoff erzeugen. Auch gemäß DE-PS 3 106 449 werden wertvolle Überzugsmitteldispersionen für Arzneimittel erhalten, die säurelösliche Polymerisate auf Basis von Alkylestern und bestimmten Aminoalkylestern der Acryl- und/oder Methacrylsäure enthalten. Unabhängig vom pH-Wert lösliche oder quellbare Polymerisate, die quartäre Ammoniumgruppen enthalten, sind in dispergierter Form zur Bildung von Arzneimittelüberzügen aus EP-A 181 515 bekannt.

Die Überzugsmitteldispersionen werden meistens unmittelbar durch Emulsionspolymerisation der entsprechenden Acryl- und/oder Methacrylmonomeren erzeugt. Es ist jedoch auch möglich, stabile Überzugsmitteldispersionen aus pulverförmigen Polymerisaten herzustellen, wenn sie durch salzartige Gruppen selbstemulgierende Eigenschaften haben; so werden gemäß DE-OS 32 08 791 sprühgetrocknete carboxylgruppenhaltige Emulsionspolymerisatpulver durch Alkalizusatz zu stabilen wäßrigen Überzugsmitteln dispergiert. Quartäre Ammoniumgruppen enthaltende Polymerisate lassen sich gemäß EP-A 181 515 in Wasser stabil dispergieren.

Das dispergierte Polymerisat ist als filmbildend zu bezeichnen, wenn es unter den Bedingungen, unter denen feste Arzneiformen mit bekannten Beschichtungsverfahren überzogen werden, zu einem geschlossenen Film auftrocknet. Die Trocknungstemperatur liegt in der Regel zwischen 10 und 80 Grad C; daher soll die nach DIN 53 787 bestimmte Mindestfilmbildungstemperatur in diesem Bereich, vorzugsweise nicht über 30 Grad C liegen. Der Anteil des Polymerisats in dem Überzugsmittel liegt in der Regel zwischen 5 und 50 Gew.-%, vorzugsweise zwischen 10 und 20 Gew.-%.

Typische Polymerisate für die Zwecke der Erfindung sind Homo- oder Copolymerisate von Acrylmonomeren. Sie können neutral sein und ganz oder nahezu ganz aus Alkylestern der Acryl- und/oder Methacrylsäure aufgebaut sein. Polymerisate, die in Wasser oder in den sauren oder schwach alkalischen Flüssigkeiten des Magen-Darm-Traktes löslich oder quellbar sind, enthalten Monomereinheiten mit polaren Gruppen, meist als Comonomer-Einheiten neben unpolaren Einheiten, wie den Alkylestern der Acryl- und/oder Methacrylsäure. Die erfindungsgemäß eingesetzten Polymerisate sind daher vorzugsweise zu 30 bis 70 Gew.-% aus Acryl- und/oder Methacrylsäure, oder zu 5 bis 50 Gew.-% aus deren Aminoalkylestern oder ganz oder zum restlichen Teil aus Alkylestern der Acryl- und/oder Methacrylsäure aufgebaut. Gegebenenfalls können bis zu 50 Gew.-% an anderen, damit radikalisch copolymersierbaren äthylenisch ungesättigten Monomeren am Aufbau der Polymerisate beteiligt sein. Unter den Alkylestern sind diejenigen mit 1 bis 4 Kohlenstoffatomen im Alkylrest bevorzugt, wozu noch Alkylsubstituenten mit 1 bis 4 Kohlenstoffatomen am Aminrest von Aminoalkylestern kommen können. Aus pharmakologischer Sicht sind Ethylacrylat und Methylmethacrylat die bestgeeigneten Esterkomponenten.

Die Zusammensetzung des Polymerisats sollte so gewählt werden, daß der daraus gebildete Film zwar trocken und hart, d.h. nicht klebrig, aber auch nicht spröd ist. Dieses Eigenschaftsbild wird erreicht, wenn die dynamische Glastemperatur des Polymerisatfilms (auch als $T_{max}$ oder $T_{g(dyn)}$ bezeichnet) nach DIN 53 445 im Bereich von -10 bis 100 Grad C, vorzugsweise 10 bis 60 Grad C liegt.

Die dynamische Glastemperatur läßt sich durch den Aufbau des Polymerisats oder, wenn Mischungen mehrerer Polymerisate eingesetzt werden, durch den Aufbau und die Mengenanteile der einzelnen Polymerisate auf den gewünschten Wert einstellen. Bekanntlich wirken sich Acrylester und höhere Methacrylester als Comonomere erniedrigend und Acryl- oder Methacrylsaure sowie niedere Methacrylester erhöhend auf die dynamische Glastemperatur aus. Der Anteil der Acryl- und/oder Methacrylsäure richtet sich in bekannter Weise nach den geforderten Löslichkeitseigenschaften oder der Diffusionsdurchlässigkeit des Überzugsfilmes. Der verbleibende Anteil der Monomeren wird dann so aus den hart- und weichmachenden Monomeren gewählt, daß die gewünschte dynamische Glastemperatur erreicht wird.

Die Filmhärte kann gewünschtenfalls auch durch Weichmachungsmittel herabgesetzt bzw. Elastizität und Dehnbarkeit entsprechend erhöht werden. Geeignete Weichmacher sind z.B. Polyethylenglykole, Triacetin und Citronensäureester.

Das Polymerisat wird in der Regel durch anionische und/oder nichtionische wasserlösliche Emulgatoren mit HLB-Werten im Bereich von 10 bis 20 stabilisiert. Ihr Anteil an dem Überzugsmittel kann bis zu 10 Gew.-% betragen. Gebräuchliche wasserlösliche Emulgatoren sind z.B. Natriumlaurylsulfat und Polyoxyethylen-sorbitan-fettsäureester.

Zusammensetzung und Eigenschaften des Überzugsmittels

Das wäßrige Überzugsmittel kann allein aus dem in der Wasserphase dispergierten Polymerisat und dem lipophilen Emulgator bestehen und ergibt klare Filme, deren Löslichkeit und Permeabilität von der Zusammensetzung des Polymers abhängen. Sie können im Magensaft löslich oder unlöslich und im wesentlichen diffusionsdurchlässig sein, aber mit ansteigendem pH-Wert im Darmsaft löslich oder quellbar werden. Das Freigabeverhalten für den eingeschlossenen Wirkstoff wird durch den Gehalt an dem lipophilen Emulgator nicht merklich verändert.

Häufig ist es erwünscht, deckende farbige Überzüge zu erzeugen. In diesem Falle können in das Überzugsmittel übliche Pigmente und gegebenenfalls Füllstoffe eingearbeitet werden. Obwohl erfindungsgemäß keine Zusätze als Gleit- und Trennmittel während des Beschichtungsverfahrens erforderlich und vorzugsweise auch nicht enthalten sind, ist der Zusatz entsprechender Stoffe, wie Kieselsäure oder Talkum, sofern er aus anderen Gründen erwünscht sein sollte, nicht grundsätzlich ausgeschlossen.

Weiße oder farbige Pigmente, wie Titandioxid, Eisenoxidfarben oder Aluminiumoxidlacke, werden in der Regel in Mengen von 10 bis 300 Gew.-%, bezogen auf das Polymerisat, verwendet. Sie können als solche oder in Form einer Aufschlemmung in Wasser mit der Dispersion des Polymerisats vermischt werden. Der lipophile Emulgator kann vor dem Vermischen der Polymerisatdispersion oder der Pigmentaufschlemmung oder beiden Komponenten anteilweise zugesetzt werden. Zur Stabilisierung der Pigmentaufschlemmung werden bis zu etwa 5 Gew.-%, berechnet auf Pigmentgewicht, des lipophilen Emulgators verwendet; sein HLB-Wert sollte vorzugsweise im oberen Teil des beanspruchten Bereichs von 3,5 bis 7 liegen.

Weitere gebräuchliche Zusätze, die auch in den erfindungsgemäßen Überzugsmitteln mitverwendet werden können, sind lösliche Farbstoffe aus der Gruppe der zulässigen Lebensmittelfarbstoffe, Glanzmittel, wie Polyethylenglykole oder Wachse, Antischaummittel, Stabilisatoren, Verdickungsmittel u.ä.

Anwendung der Überzugsmittel

Das erfindungsgemäß zusammengesetzte, gewünschtenfalls mit Zusätzen der angegebenen Art versehene Überzugsmittel ist unter normalen Umgebungsbedingungen, d.h. im geschlossenen Behälter bei Temperaturen nicht über 30 Grad C, über mehrere Wochen oder Monate unverändert haltbar. Es kann daher gebrauchsfertig formuliert in den Handel gebracht, versandt und bis zum Verbrauch gelagert werden. Dadurch sind Vorrichtungen zum Homogenisieren, Stabilieren u.dergl. am Anwendungsort entbehrlich.

Oft ist es zweckmäßig, das Überzugsmittel vor dem Sprühauftrag auf eine den Sprühbedingungen angepaßte Konzentration zu verdünnen. Für die Anwendung im Dragierkessel und in Wirbelschichtgeräten sind Polymerisatgehalte von 5 bis 25 Gew.-% zweckmäßig.

Die neuen Überzugsmittel eignen sich in gleicher Weise wie herkömmliche wäßrige Überzugsmitteldispersionen zum Beschichten von festen Arzneimitteln. Bevorzugt werden kompakte Arzneiformen beschichtet, die keine wesentlichen inneren Hohlräume enthalten; dazu zählen Komprimate (Tabletten), Dragees, Pillen, Granulate, Pellets, Partikel, Kristalle. Übliche Schichtdicken liegen zwischen 2 und 500, vorzugsweise 10 bis 200 Mikrometer. Man erhält magensaftresistente Überzüge, die sich je nach der Zusammensetzung im neutralen oder schwach alkalischen Milieu des Darmsaftes lösen oder zumindest unter Quellung diffusionsdurchlässig werden. Man kann auch Pulver damit granulieren und gegebenenfalls das erhaltene Granulat zu Tabletten verpressen.

**Beispiel 1:**

Sprühsuspension für Retardüberzüge

Eine Suspension aus 7,1 g Glycerin-monostearat (HLB = 3,5) und 516,7 g Wasser wird unter Rühren auf 65-70 Grad C erhitzt, mit einem schnellaufenden Rührwerk (Ultra-Turrax) homogenisiert und anschließend unter Rühren auf 20-30 Grad C abgekühlt.

Zu dieser Suspension gibt man unter Rühren 476,2 g einer 30%-igen wäßrigen, anionischen Dispersion eines Emulsionspolymerisats aus 67 Gew.-% Ethylacrylat und 33 Gew.-% Methylmethacrylat (Handelsbezeichnung EUDRAGIT NE30D, Röhm GmbH, Darmstadt). Die erhaltene Sprühsuspension hat einen Feststoffgehalt von 15 Gew.-%, eine Viskosität von etwa 500 mPa s. Sie ist gebrauchsfertig und lagerfähig.

393,75 g der Sprühsuspension werden im Wirbelschichtgerät auf 0,75 kg Verapamil-HCl-Pellets (0,3-1,25 mm, Wirkstoffgehalt 88 Gew.-%) bei einer Zulufttemperatur von 40 Grad C mit einer Sprühgeschwindigkeit von 10 g/min aufgetragen. Anschließend wird 2 Stunden bei 40 Grad C getrocknet.

Die überzogenen Pellets enthalten 7,5 Gew.-% Überzugsmaterial. Sie zeigen in vitro eine über 8 Stunden verzögerte Wirkstoffabgabe.

**Beispiel 2:**

Sprühsuspension für Retardüberzüge

Eine Mischung aus 6,0 g Glycerin-monostearat und 570 g Wasser wird unter Rühren auf 65-70 Grad C erhitzt, nach Zugabe von 24 g Triethylcitrat mit einem schnellaufenden Rührwerk (Ultra-Turrax) homogenisiert und anschließend unter Rühren auf 20-30 Grad C abgekühlt.

Zu dieser Suspension gibt man unter Rühren 320 g einer 30%igen wäßrigen Dispersion eines redispergierten Copolymerisats aus 65 Gew.-% Methylmethacrylat, 30 Gew.-% Ethylacrylat und 5 Gew.-% Trimethylammoniumethylmethacrylat-chlorid sowie 80 g einer 30%-igen wäßrigen Dispersion eines redispergierten Copolymerisats aus 60 Gew.-% Methylmethacrylat, 30 Gew.-% Ethylacrylat und 10 Gew.-% Trimethylammoniumethylmethacrylat-chlorid (Handelsbezeichnungen EUDRAGIT RS30D und RL30D, Röhm GmbH, Darmstadt). Man erhält eine lagerfähige und gebrauchsfertige Sprühsuspension mit einem Feststoffgehalt von 15 Gew.-% und einer Viskosität von etwa 500 mPa s.

875 g der Sprühsuspension werden im Wirbelschichtgerät auf 0,75 kg Theophyllin-Granulat (0,3-0,8 mm) bei einer Zulufttemperatur von 40 Grad C mit einer Sprühgeschwindigkeit von 10 g/min aufgetragen. Anschließend wird 24 Stunden bei 40 Grad C im Umlufttrockenschrank getrocknet.

Die überzogenen Pellets enthalten 14 Gew.-% Überzugsmaterial. Sie zeigen in vitro eine über 8 Stunden verzögerte Wirkstoffabgabe.

**Beispiel 3:**

Sprühsuspension für magensaftresistente Überzüge

Eine Mischung aus 3,7 g Glycerin-monostearat und 465 g Wasser wird unter Rühren auf 65-70 Grad C erhitzt, nach Zugabe von 24,4 g Triethylcitrat mit einem schnellaufenden Rührwerk (Ultra-Turrax) homogenisiert und anschließend unter Rühren auf 20-30 Grad C abgekühlt.

Zu dieser Suspension gibt man unter Rühren 406,5 g einer 30%-igen wäßrigen Dispersion eines Emulsionspolymerisats aus 50 Gew.-% Methyacrysäure und 50 Gew.-% Ethylacrylat (Handelsbezeichnung EUDRAGIT L30D, Röhm GmbH, Darmstadt). Man erhält eine gebrauchsfertige und lagerfähige Sprühsuspension mit einem Feststoffgehalt von 15 Gew.-% und einer Viskosität von etwa 500 mPa s.

1082,4 g der Sprühsuspension werden im rotierenden Dragierkessel bei einer Zulufttemperatur von 50 Grad C mit einer Sprühgeschwindigkeit von 8-10 g/min auf 2,8 kg Placebotabletten aufgetragen. Anschließend wird 2 Stunden bei 40 Grad C im Umlufttrockenschrank getrocknet.

Die überzogenen Tabletten enthalten 6 mg/qcm Überzugsmaterial. Sie zerfallen in künstlichem Darmsaft von pH 6,8 innerhalb von 30 min.

**Beispiel 4:**

Sprühsuspension für geschmackisolierende Überzüge

Eine Mischung aus 4 g Glycerin-monostearat, 213,3 g Wasser, 16 g Triethylcitrat und 266,7 g einer 30%-igen wäßrigen Dispersion eines dispergierten Copolymerisats aus 60 Gew.-% Methylmethacrylat, 30 Gew.-% Ethylacrylat und 10 Gew.-% Trimethylammoniumethylmethacrylat-chlorid (Handelsbezeichnung EUDRAGIT RL30D, Röhm GmbH, Darmstadt) wird unter Rühren auf 65-70 Grad C erhitzt, mit einem schnellaufenden Rührwerk (Ultra-Turrax) homogenisiert und anschließend unter Rühren auf 20-30 Grad C abgekühlt. Die erhaltene Sprühsuspension hat einen Feststoffgehalt von 20 Gew.-%, eine Viskosität von etwa 500 mPa s und ist gebrauchsfertig und lagerfähig.

137,5 g der Sprühsuspension werden mit der gleichen Menge Wasser verdünnt und im rotierenden Dragierkessel bei einer Zulufttemperatur von 50 Grad C mit einer Sprühgeschwindigkeit von 8-10 g/min auf 2,8 kg Verapamil-HCl-Tabletten aufgetragen. Anschließend wird 24 Stunden bei 40 Grad C im Umlufttrockenschrank getrocknet.

Die überzogenen Tabletten enthalten 1 mg/qcm Überzugsmaterial. Sie bleiben etwa 1 Minute geschmackisoliert und geben in vitro mehr als 80 % des Wirkstoffes innerhalb von 15 min ab. (US-P XXI, Meth. 2, Drehzahl: 100/min, 0,1 normale Salzsäure)

**Beispiel 5:**

Tensidhaltige Pigmentsusupension

Eine Mischung aus 8 g Glycerin-monostearat und 800 g Wasser wird unter Rühren auf 65-70 Grad C erhitzt und nach Zugabe von 32 g Triethylcitrat mit einem schnellaufenden Rührwerk (Ultra-Turrax) homogenisiert. Beim Homogenisieren gibt man 53,3 g Rotpigment (Sicopharm rot 30) und 106,7 g Titandioxid zu. Anschließend wird unter Rühren auf 20-30 Grad C abgekühlt.

Die Pigmentsuspension hat einen Feststoffgehalt von 20 Gew.-% und sedimentiert während längerer Lagerung nur geringfügig. Sie kann mit wäßrigen Überzugsmitteldispersionen zu Sprühsuspensionen abgemischt werden. Dazu verdünnt man 245,5 g der Pigmentsuspension mit 458,5 g Wasser und gibt sie unter Rühren zu 131 g der in Beispiel 4 verwendeten Dispersion (EUDRAGIT RL30D).

884 g der Suspension werden im rotierenden Dragierkessel bei einer Zulufttemperatur von 50 Grad C mit einer Sprühgeschwindigkeit von 11 g/min auf 5 kg Placebotabletten aufgetragen. Anschließend wird 24 Stunden bei 40 Grad C im Umlufttrockenschrank getrocknet.

Die überzogenen Tabletten enthalten 1 mg/qcm Überzugsmaterial. Sie zerfallen in vitro innerhalb von etwa 2 min.

**Beispiel 6:**

Tensid-Dispersion zur Verringerung des Verklebens

Eine Mischung aus 10 g Glycerin-monostearat und 950 g Wasser wird unter Rühren auf 65-70 Grad C erhitzt und mit einem schnellaufenden Rührwerk (Ultra-Turrax) homogenisiert. Anschließend wird unter Rühren auf 20-30 Grad C abgekühlt. Die Tensiddispersion ist damit gebrauchsfertig. Sie enthält 1 Gew.-% Feststoff.

88 g der Tensiddispersion werden im Wirbelschichtgerät bei einer Zulufttemperatur von 40 Grad C mit einer Sprühgeschwindigkeit von 10 g/min auf 0,881 kg des nach Beispiel 2 hergestellten Theophyllin-Granulats aufgetragen. Anschließend wird 24 Stunden bei 40 Grad C im Umlufttrockenschrank getrocknet.

Das überzogenen Granulat mit 14 Gew.-% Lackauftrag und anschließendem Auftrag von 0,1 Gew.-% Glycerin-monostearat zeigt keinerlei Klebrigkeit mehr und erlaubt eine langfristige Lagerung.

**Beispiel 7:**

Sprühsuspension für schnell zerfallende Überzüge

Man verfährt zur Herstellung einer Sprühsuspension wie im Beispiel 4, setzt jedoch anstelle von Glycerin-monostearat die gleiche Menge an zweifach oxethyliertem Stearylalkohol (HLB = 4,9) ein. 137,5 g der Sprühsuspension werden im rotierenden Dragierkessel bei einer Zulufttemperatur von 50 Grad C mit einer Sprühgeschwindigkeit von 8-10 g/min auf 2,8 kg Placebo-Tabletten aufgetragen. Anschließend wird 24 Stunden bei 40 Grad C im Umlufttrockenschrank getrocknet. Die Tabletten haben Zerfallszeiten von 2-5 min.

**Beispiele 8 und 9:**

Sprühsuspension für schnell zerfallende Überzüge

Man verfährt wie im Beispiel 7, setzt jedoch anstelle von zweifach oxethyliertem Stearylalkohol die gleiche Menge an Sorbitan-monostearat (Handelsbezeichnung SPAN 80, HLB = 4,7) bzw. an Glycerin-monolaurat (HLB = 5,6) ein. Man erhält in beiden Fällen Tabletten mit Zerfallszeiten von 2-5 min.

**Beispiel 9:**

Vergleich der erfindungsgemäßen Arbeitsweise (A) mit der Arbeitsweise mit Talkum als Trennmittel (B) und ohne Trennmittel (C)

A. Eine Mischung aus 2,4 g Glycerin-monostearat, 19,1 g Triethylcitrat und 982 g Wasser wird nach Zugabe von 0,1 g 33 %igem 20-fach oxethyliertem Sorbitan-monooelat (Tween 80, HLB = 11,3) mit einem schnell laufenden Rührwerk homogenisiert. Dabei werden 47,8 g Braunpigment (Sicopharm braun 70) sowie 47,8 g Titandioxid zugegeben. Diese Pigmentsuspension sedimentiert praktisch nicht.

Sie wird unter Rühren zu 318 g der im Beispiel 4 verwendeten Dispersion zugemischt. 1417 g der erhaltenen Suspension werden im rotierenden Dragierkessel bei einer Zulufttemperatur von 50 Grad C mit einer Sprühgeschwindigkeit von 24 g/min auf 10 kg Placebotabletten aufgetragen. Anschließend wird 24 Stunden bei 40 Grad C im Umlufttrockenschrank getrocknet. Der Lackauftrag beträgt 1 mg/qcm. Der Überzug ist glatt, glänzend und gleichmäßig. Die Zerfallszeiten der überzogenen Tabletten liegen zwischen etwa 2 und 5 min. Sie bleiben nach einer Lagerung von 2 Monaten unverändert.

B. Eine Aufschlemmung von 45 g Talkum in 210 g Wasser wird nach Zugabe von 9 g Polyethylenglykol (MW = 6000) mit einem schnell laufenden Rührwerk (Ultra Turrax) homogenisiert. Dabei werden 12 g Gelblack E 104 und 24 g Titandioxid zugesetzt. Die Pigmentsuspension sedimentiert innerhalb 30 min vollständig.

Zur Herstellung einer sprühbaren Pigmentsuspension werden 300 g der erhaltenen 30 %-igen Suspension mit 6 g Triethylcitrat versetzt, mit 144 g Wasser verdünnt und mit 100 g der im Beispiel 4 verwendeten Dispersion vermischt. Die erhaltene Suspension sedimentiert nach kurzer Zeit.

550 g der frisch homogenisierten Suspension werden im rotierenden Dragierkessel bei einer Zulufttemperatur von 50 Grad C mit einer Sprühgeschwindigkeit von 8 g/min auf 3 kg Placebotabletten aufgetragen. Anschließend wird 24 Stunden bei 40 Grad C im Umlufttrockenschrank getrocknet. Der Lackauftrag beträgt 1 mg/qcm. Der Überzug ist glatt, glänzend und gleichmäßig. Die Tabletten haben Zerfallszeiten von 2-5 min.

C. Ein Mischung aus 19,1 g Triethylcitrat und 982 g Wasser wird mit einem schnell laufenden Rührwerk (Ultra Turrax) homogenisiert. Dabei werden 47,8 g Braunpigment (Sicopharm braun 70) und die gleiche Menge Titandioxid zugesetzt. Diese Pigmentaufschlemmung gibt man zu 318 g der im Beispiel 4 eingesetzten Dispersion.

1415 g der erhaltenen Suspension werden im rotierenden Dragierkessel bei einer Zulufttemperatur von 50 Grad C mit einer Sprühgeschwindigkeit von 17 g/min auf 10 kg Placebotabletten aufgetragen. Trotz reduzierter Sprühgeschwindigkeit zeigt der Versuch während des Auftrags deutliche Anzeichen von Verklebungen.

Anschließend wird 24 Stunden bei 40 Grad C im Umlufttrockenschrank getrocknet. Der Lackauftrag beträgt 1 mg/qcm. Der Überzug ist matt, uneben und ungleichmäßig. Die Zerfallszeiten der überzogenen Tabletten liegen zwischen etwa 1 und 10 min.

## Patentansprüche

1. Aus pharmakologisch unbedenklichen Bestandteilen aufgebautes filmbildendes wäßriges Überzugsmittel für feste Arzneiformen, enthaltend
   a) ein filmbildendes dispergiertes Polymerisat auf Basis von Acryl- und/oder Methacrylsäure oder deren Aminoalkylestern und/oder deren Alkylestern,
   b) einen wasserunlöslichen organischen lipophilen Emulgator mit einem HLB-Wert zwischen 3,5 und 5 in einer Menge von mindestens 0,1 Gew.-%, jedoch weniger als 10 Gew.-%, bezogen auf das Polymerisat.

2. Überzugsmittel nach Anspruch 1, dadurch gekennzeichnet, daß es zusätzlich wenigstens ein Pigment enthält.

3. Überzugsmittel nach Anspruch 2, dadurch gekennzeichnet, daß es mindestens 2 Gew.-%, jedoch weniger als 10 Gew.-%, bezogen auf das Polymerisat, des lipophilen Emulgators enthält.

4. Verfahren zur Herstellung eines Überzugsmittels gemäß Anspruch 2, dadurch gekennzeichnet, daß eine wäßrige Dispersion des Emulsionspolymerisats mit einer wäßrigen Mischung des Pigments und eines lipophilen Emulgators mit einem HLB-Wert zwischen 3,5 und 5 in einer Menge von mindestens 0,1 jedoch weniger als 10 Gew.-%, bezogen auf das Polymerisat, vermischt wird.

5. Verfahren nach Anspruch 4, dadurch gekennzeichnet, daß eine wäßrige Dispersion des Emulsionspolymerisats mit einem Gehalt eines lipophilen Emulgators mit einem HLB-Wert zwischen 3,5 und 5 eingesetzt wird.

6. Verwendung des Überzugsmittels nach einem oder mehreren der Ansprüche 1 bis 3 zum Überziehen von Arzneiformen.

7. Verwendung gemäß Anspruch 6 nach dem Wirbelbettverfahren.

8. Verwendung gemäß Anspruch 6 oder 7, dadurch gekennzeichnet, daß Pulver, Kristalle, Granulate oder Komprimate von Arneistoffen oder solche enthaltenden Feststoffmischungen granuliert und überzogen werden.

## Claims

1. A film-forming aqueous coating substance for solid pharmaceutical compositions synthesised from pharmacologically harmless constituents, containing
   a) a film-forming dispersed polymer on the basis of acrylic and/or methacrylic acid or the amino alkyl esters thereof and/or the alkyl esters thereof,
   b) a water-insoluble organic lipophilic emulsifier with an HLB-value between 3.5 and 5, in an amount of at least 0.1 wt.%, but less than 10 wt.%, based on the polymer.

2. A coating substance according to claim 1, characterised in that it contains additionally at least one pigment.

3. A coating substance according to claim 2, characterised in that it contains at least 2 wt.%, but less than 10 wt.%, based on the polymer, of lipophilic emulsifier.

4. A process for preparing a coating substance according to claim 2, characterised in that an aqueous dispersion of the emulsion polymer is mixed with an aqueous mixture of the pigment and a lipophilic emulsifier having an HLB-value between 3.5 and 5, in an amount of at least 0.1, but less than 10 wt.%, based on the polymer.

5. A process according to claim 4, characterised in that an aqueous dispersion of the emulsion polymer containing a lipophilic emulsifier with an HLB-value of between 3.5 and 5 is used.

6. Use of the coating substance according to one or more of claims 1 to 3, for coating pharmaceutical compositions.

7. Use as claimed in claim 6, according to the fluid-bed process.

8. Use according to claim 6 or 7, characterised in that powders, crystals, granules or compressed preparations of pharmaceutical substances or solid mixtures containing such pharmaceutical substances are granulated and coated.

## Revendications

1. Matière de revêtement aqueuse filmogène pour des formes médicamenteuses solides, composée de constituants inoffensifs du point de vue pharmacologique, contenant
a) un polymère filmogène dispersé, à base d'acides acrylique et/ou méthacrylique ou d'esters aminoalkyliques de ceux-ci et/ou d'esters alkyliques de ceux-ci,
b) un émulsionnant lipophile organique insoluble dans l'eau, ayant un indice HLB (indice d'hydrophilie-lipophilie) compris entre 3,5 et 5, dans une proportion d'au moins 0,1% en poids, mais inférieure à 10% en poids, par rapport au polymère.

2. Matière de revêtement selon la revendication 1, caractérisée en ce qu'elle contient en plus au moins un pigment.

3. Matière de revêtement selon la revendication 2, caractérisée en ce qu'elle contient au moins 2% en poids, mais moins de 10% en poids, par rapport au polymère, de l'émulsionnant lipophile.

4. Procédé de préparation d'une matière de revêtement selon la revendication 2, caractérisé en ce qu'une dispersion aqueuse du produit de polymérisation en émulsion est mélangée avec un mélange aqueux du pigment et d'un émulsionnant lipophile ayant un indice HLB compris entre 3,5 et 5, dans une proportion d'au moins 0,1% en poids, mais inférieure à 10% en poids, par rapport au produit de polymérisation.

5. Procédé selon la revendication 4, caractérisé en ce qu'on utilise une dispersion aqueuse du produit de polymérisation en émulsion qui contient un émulsionnant lipophile ayant un indice HLB compris entre 3,5 et 5.

6. Utilisation de la matière de revêtement selon l'une quelconque des revendications 1 à 3 pour l'enrobage de formes médicamenteuses.

7. Utilisation selon la revendication 6 suivant le procédé en lit fluidisé.

8. Utilisation selon la revendication 6 ou 7, caractérisée en ce que des poudres, des cristaux, des granulés ou des comprimés de substances médicamenteuses ou des mélanges de matières solides contenant de telles substances, sont granulés et enrobés.